# EUROPEAN PATENT APPLICATION

(11) **EP 4 456 083 A1**
(43) Date of publication of application: **30.10.2024**
(21) Application number: 24171741.2
(22) Date of filing: 22.04.2024
(51) Int. Cl.: G16H 40/63, G16H 20/40, G16H 30/20, G16H 30/40, G16H 50/70, A61B 1/00

(54) **CORRECTIVE ADJUSTMENT OF IMAGE PARAMETERS USING ARTIFICIAL INTELLIGENCE**

(30) Priority: 24.04.2023 US 202318138328
(71) Applicant: Karl Storz SE & Co. KG, 78532 Tuttlingen (DE)
(72) Inventor: Hale, Eric, 78532 Tuttlingen (DE)

(57) **Abstract**

Systems and methods are provided that enable contextual correction functions based on a current step in a surgery or surgical procedure and based on what is displayed on a display. By using information about what is depicted on the display and the current step of the surgery or surgical procedure, a controller can implement data models that automatically perform correction functions without the need for physician input.

## Description

### BACKGROUND

Endoscopes can be used to provide a visualization of a surgical scene during the course of a surgery or surgical procedure. Video endoscopes or endoscopes used in conjunction with camera heads can provide a video stream of this visualization through the use of associated image sensors. The endoscope and/or an associated camera head often includes optical components, such as beam splitters, filters, and focusing elements, that assist with conditioning light from the surgical scene such that the associated image sensor or sensors are able to generate a video feed of the surgical scene. Image sensors together with a prism assembly comprising one or more beam splitters may form an imaging assembly.

Operating rooms may be used to perform one or more surgeries or surgical procedures. The surgery or surgical procedure may follow a set workflow, so that the same surgery or surgical procedure is uniformly performed even with different patients, different surgeons, and/or different surgical assistants. Over the course of time, a physician may learn the nuanced requirements of the surgery or surgical procedure, leading to improved patient outcomes. The surgeon may nonetheless need to handle one or more surgical instruments or perform other tasks within the operating room that negatively impacts the surgeon's performance and delays the surgery or surgical procedure, such as adjusting imaging equipment.

### SUMMARY

Issues associated with the above are addressed with systems and methods disclosed herein. By providing one or more data models (e.g., data models based on Artificial Intelligence (AI) and/or Machine Learning (ML)) that can perform corrective functions during the course of a surgery or surgical procedure, the present disclosure beneficially enables one or more corrective actions to be automatically performed. The data models may be used, for example, to automatically perform a white balancing, to automatically adjust the focus of one or more imaging devices, to cause an illumination device to emit light, and the like at an appropriate time during a surgery or surgical procedure.

Additionally, the systems and methods discussed herein may include multiple different data models, with each data model trained to handle one or more corrective actions. For example, a first data model may be trained to perform a white balancing, while a second data model may be trained to adjust an intraoperative image (e.g., using image processing). A controller may operate the first and second data model, such that the controller determines, based on timing information and readings from one or more sensors, that a corrective action needs to be taken. The controller may then use an appropriate data model to perform the corrective action.

### BRIEF DESCRIPTION OF THE DRAWINGS

Fig. 1 illustrates aspects of an endoscope according to at least one exemplary embodiment;
Fig. 2A illustrates a block diagram of a system according to at least one exemplary embodiment;
Fig. 2B illustrates aspects of an artificial intelligence according to at least one exemplary embodiment;
Fig. 3 illustrates a method according to at least one exemplary embodiment; and
Fig. 4 illustrates a method according to at least one exemplary embodiment.

### DETAILED DESCRIPTION

Embodiments of the present disclosure will be described in connection with components and features of an endoscope and data models used to perform adjustments to image parameters. However, to avoid unnecessarily obscuring the present disclosure, the description omits a number of known structures and devices. These omissions are not to be construed as limitations of the scope of the claimed disclosure. Specific details are set forth to provide an understanding of the present disclosure. It should, however, be appreciated that the present disclosure may be practiced in a variety of ways beyond the specific detail set forth herein.

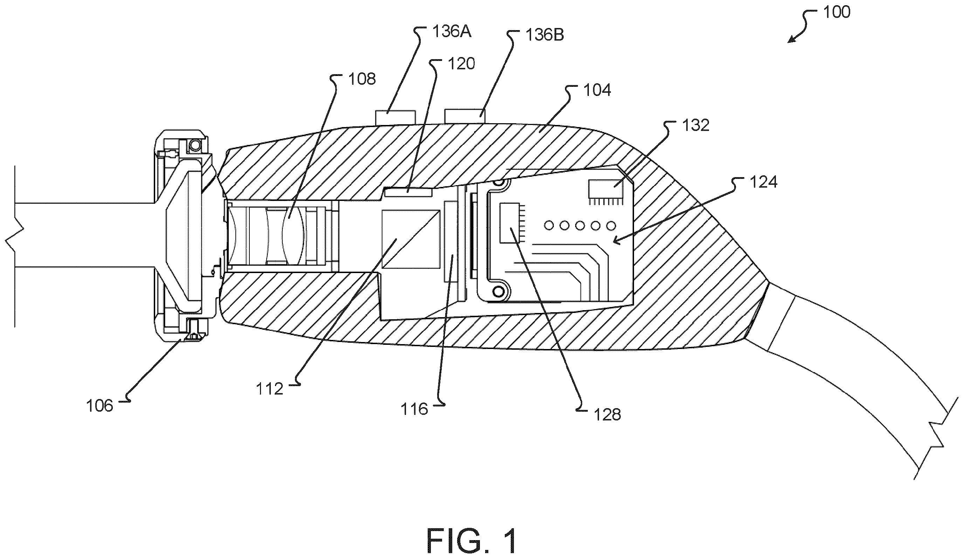

Turning first to Fig. 1, aspects of an endoscopic camera head 100 are shown in accordance with at least one exemplary embodiment of the present disclosure. It should be noted that this disclosure will generally refer to system 100 as an endoscopic camera head, however, it should be understood that the camera head presented may also be a portion of a single unit endoscope and camera system, usually called a video endoscope, a portion of a surgical scope, or as a camera head or camera element acting as part of, or attached to, an exoscope. The endoscopic camera head 100 includes a housing 104 within or whereon are housed an optical assembly 108, a prism or prism assembly 112, a first image sensor 116, a second image sensor 120, and processing electronics 124. The processing electronics 124 includes a controller 128 and an orientation/movement detector 132, such as a gyroscope or an accelerometer. The endoscopic camera head 100 also includes a grasping mechanism 106, such as a bayonet connection, disposed on a distal end thereof, as well as one or more buttons 136A-136B that enable a user to interact with the endoscope 100 or one or more components thereof. In some embodiments, the endoscopic camera head 100 may include additional or alternative components, and the components illustrated in Fig. 1 are in no way limiting. For example, the endoscopic camera head 100 may include additional sensor modules, with the additional sensor modules capable of receiving, for example, different wavelengths, polarizations, magnifications, or intensities of light. In another example, the controller 128 may be disposed externally from the endoscopic camera head 100. In such cases, the controller 128 may communicate with the endoscopic camera head 100 (or components thereof) wirelessly and/or through a wired connection.

The housing 104 may enable storage of one or more hardware components (e.g., cables/wiring, batteries, optical fibers, etc.) that enable one or more additional functions of the endoscopic camera head 100, such as illumination from the distal end of an attached endoscope, video recording of a surgical site, image processing, combinations thereof, and the like.

The grasping mechanism 106 may enable the endoscopic camera head 100 to be connected or attached to one or more other surgical components, usually to a detachable endoscope or components of an exoscope which capture light from a surgical site and relay the captured light to the optical assembly 108 of the endoscopic camera head 100. In some embodiments, the grasping mechanism 106 may include, or allow attachment to, additional optical components, such as spectral filters that filter light entering the optical assembly 108.

The optical assembly 108 may be or comprise one or more mirrors, lenses, filters, polarizers, beam splitters, prisms, and/or windows capable of conditioning and/or directing received light to one or more other optical components in the endoscopic camera head 100, such as focusing and directing to the prism 112. The optical assembly 108 may be disposed closer to the distal end of the endoscopic camera head 100 than the other optical components, such that the received light passes through the optical assembly 108 before entering the other optical components such as the prism 112. In some embodiments, the optical assembly 108 may be configured to filter, adjust, bend, focus, or otherwise alter the received light based on the type of light received, the type of surgery or surgical procedure in which the endoscopic camera head 100 is used, combinations thereof, and the like. For example, some endoscopes may include a spectral filter in the optical assembly 108 that filters out light of a specific wavelength or a range of wavelengths.

The prism 112 may function as a beam splitter that separates the light received from the optical assembly 108 into two or more light paths. For example, as illustrated in Fig. 1, the prism 112 may separate the received light into two different light paths, with a first light path directed toward the first image sensor 116, and a second light path directed toward the second image sensor 120. The prism 112 may be capable of splitting the received light based on the different wavelengths of the light. In one embodiment, the prism 112 may be able to separate the received light into a white light signal (e.g., light with wavelengths between about 400 nanometers (nm) and about 700 nm) and an infrared (IR) signal (e.g., light with wavelengths above about 800 nm).

The first image sensor 116 and the second image sensor 120 may be capable of capturing the light split by the prism 112 and converting the light into an electric signal. The image sensors, such as a CMOS or CCD sensor, include a plurality of pixels, each containing a photodetector that converts detected light into an electric signal. The number and orientation of the pixels is not limited, and the plurality of pixels may be disposed in, for example, an array. The electric signal may be passed from the first image sensor 116 and/or the second image sensor 120 to the processing electronics 124 and/or the controller 128, where the signal is processed to produce individual images or a video stream. In some embodiments, the image sensors may be capable of processing light with different wavelengths, such as when the endoscope 100 captures a fluorescence image of Indocyanine Green (ICG) fluorophores. In one embodiment, the first image sensor 116 may be capable of processing white light or spectra thereof (e.g., red light, blue light, green light, etc.), and as such, its corresponding image sensor may comprise a Bayer, or other appropriate color filter, while the second image sensor 120 may be capable of processing IR or near-IR light (e.g., light with wavelengths above about 800 nm), and thus its corresponding image sensor may contain no corresponding color filter, increasing, thereby the sensitivity of the second image sensor relative to the first. In embodiments where the endoscopic camera head 100 includes additional sensors, each sensor may receive and process different wavelengths of light. For example, the white light may be further split into three separate channels of red light, blue light, and green light, with each different color received by a separate image sensor.

The controller 128 may communicate with one or more components of the endoscopic camera head 100 (e.g., the first image sensor 116, the second image sensor 120, the processing electronics 124, etc.) to permit the endoscopic camera head 100 and/or components thereof to be controlled or otherwise operated. While the controller 128 is illustrated as being disposed within the endoscope 100 in Fig. 1, it is to be understood that, in other embodiments, the controller 128 may be disposed in alternative components, such as within a computer disposed within the operating room. The processing electronics 124 may include one or more electrical components (e.g., resistors, capacitors, transformers, batteries, wiring, cables, etc.) that can be used by the controller 128 to perform one or more functions associated with the endoscopic camera head 100. For example, the processing electronics 124 may provide a power source used by the controller 128 to power a light source in the endoscopic camera head 100.

The orientation/movement detector 132 may be or comprise an inertial sensor capable of capturing measurements related to acceleration, rotation, or other movement. The orientation/movement detector 132 may communicate with the processing electronics 124 and/or the controller 128. In some embodiments, the measurements generated by the orientation/movement detector 132 may be sent to and used by the controller 128 to determine a magnitude and direction in which the endoscopic camera head 100 has moved. In some embodiments, the endoscopic camera head 100 may include one or more orientation/movement detectors disposed at various locations in the overall endoscopic system, such as near the distal tip of the endoscope as well as within the endoscopic camera head 100, such that the controller 128 can receive multiple readings to determine a more accurate movement change in orientation of the endoscopic camera head 100 (or a component thereof).

The one or more buttons 136A-136B may enable the user to interact with one or more components of the endoscope 100 and/or to adjust one or more settings associated with the endoscope 100. In some embodiments, the buttons 136A-136B may enable a physician to navigate through an on-screen menu, such as a menu rendered to the display 220. The on-screen menu may be predetermined based on, for example, the type of surgery or surgical procedure. In some cases, the physician may be able to progress the surgery or surgical procedure through use of the buttons 136A-136B. For example, the surgery may have a series of predetermined steps, with the first step being a white balancing. At a first step, the physician performs a white balancing for the endoscope 100 by pointing the endoscope 100 at a white object (e.g., a piece of white paper). Once the physician has performed the white balancing, the physician may press the first button 136A to advance the surgery to a second step, where the physician can insert the endoscope 100 into the surgical site. In this example, the on-screen menu may progress through the series of predetermined steps based on the physician's inputs. During the first step, for instance, the on-screen menu may indicate to the physician that a white balancing is to be performed and may have a rendering of the current video feed of the endoscope 100, and then may indicate during the second step that the endoscope 100 is to be introduced to the surgical site and render a surgical scan of the surgical site. In other embodiments and as discussed in further detail below, the buttons 136A-136B may be connected or linked to one or more data models, such that the surgery or surgical procedure may progress automatically with limited or no input from the physician.

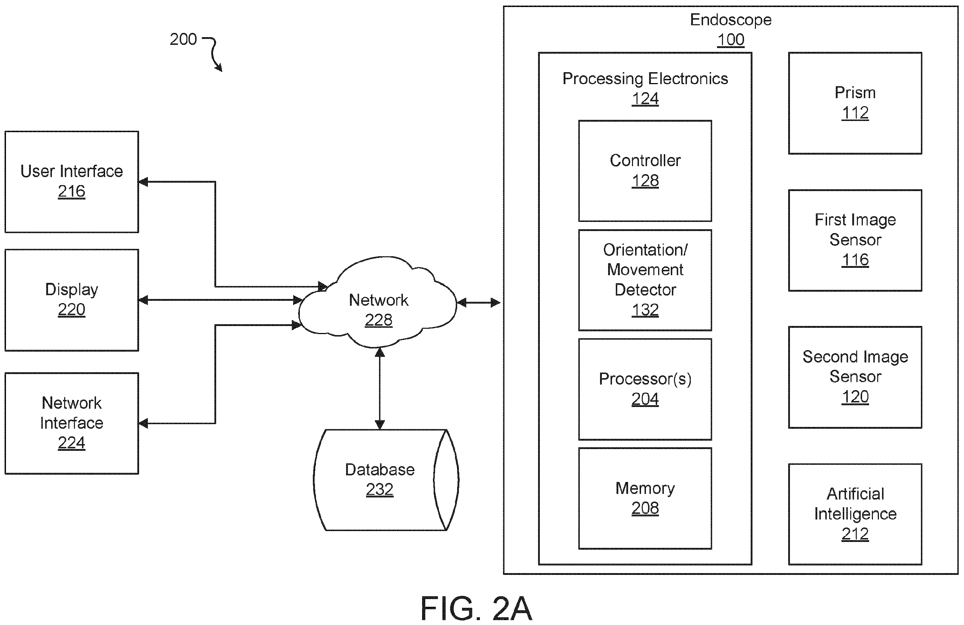

Turning next to Figs. 2A-2B, aspects of a surgical system 200 are shown in accordance with at least one exemplary embodiment. The system 200 includes the endoscopic camera head 100, a user interface 216, a display 220, a network interface 224, a network 228, and a database 232. Notwithstanding the foregoing, the system 200 may include additional or alternative components, and may also omit one or more components shown. In some embodiments, the system 200 may correspond to a processing unit to which the endoscopic camera head 100 is connected. Such an external controller may be disposed in a camera control unit (CCU).

The endoscopic camera head 100 may include the prism or prism assembly 112, the first image sensor 116, the second image sensor 120, and the processing electronics 124 as discussed with respect to Fig. 1. Additionally, the endoscopic camera head 100 may include one or more processors 204, a memory 208, and/or Artificial Intelligence (AI) 212, or some of these elements may be contained within a CCU connected thereto.

The processor 204 may provide processing functionality and may correspond to one or many computer processing devices. For instance, the processor 204 may be provided as a Field Programmable Gate Array (FPGA), an Application-Specific Integrated Circuit (ASIC), any other type of Integrated Circuit (IC) chip, a collection of IC chips, a microcontroller, a collection of microcontrollers, a GPU(s), or the like. As another example, the processor 204 may be provided as a microprocessor, Central Processing Unit (CPU), Graphics Processing Unit (GPU), Neural Processing Unit (NPU), and/or plurality of microprocessors that are configured to execute the AI 212 and/or data stored in memory 208. The processor 204 enables various functions of the endoscopic camera head 100 and/or system 200 upon executing the AI 212 and/or data stored in the memory 208.

The memory 208 may be or comprise a computer readable medium including instructions that are executable by the controller 128 and/or the processor 204. The memory 208 may include any type of computer memory device and may be volatile or non-volatile in nature. In some embodiments, the memory 208 may include a plurality of different memory devices. Non-limiting examples of memory 208 include Random Access Memory (RAM), Read Only Memory (ROM), flash memory, Electronically-Erasable Programmable ROM (EEPROM), Dynamic RAM (DRAM), etc. The memory 208 may include instructions that enable the controller 128 to control the various elements of the system 200 and to store data, for example, into the database 232 and retrieve information from the database 232. The memory 208 may be local (e.g., integrated with) the endoscopic camera head 100 or separate from the endoscopic camera head 100.

The AI 212 comprise computer-readable software that is executable by the controller 128 and/or the processor 204 that cause the controller 128 and/or the processor 204 to perform one or more functions. For example, the AI 212 may comprise feature detection algorithms (e.g., edge detection algorithms, Harris Corner Detection, Scale-Invariant Feature Transform (SIFT) algorithms, Speeded-Up Robust Features (SURF) algorithms, combinations thereof, etc.) that enable the controller 128 to determine whether an image or an image stream (e.g., a video) is misaligned (e.g., relative to a gravity vector, such as when the image captured by the endoscope 100), whether white balancing should be performed (e.g., based on analysis of the AI 212 of one or more images and/or an image stream), combinations thereof, and the like. The controller 128 may process one or more images and/or video streams using the AI 212 to determine and then perform one or more corrective actions. For example, the controller 128 may use the AI 212 to process one or more images (e.g., preoperative images or scans) and visually orient the one or more images on a display to allow a physician to better view the surgical site. It is to be understood that, while the AI 212 is depicted as being disposed within the endoscope 100, the AI 212 may be housed in a location other than the endoscope 100. For example, the AI 212 may be housed in a computing module outside of the endoscope 100. In such embodiments, the endoscope 100 may comprise electronics sufficient to capture and send images and/or a video stream to the computing module. The computing module (which may comprise the processing electronics 124 and the AI 212) may then perform one or more steps of the methods, data processing, or other functions of the processing electronics 124 and/or the AI 212 as described herein.

The user interface 216 includes hardware and/or software that enables user input to the system 200 and/or any one or more components thereof. The user interface 216 may include a keyboard, a mouse, a touch-sensitive pad, touch-sensitive buttons (e.g., the buttons 136A-136B), mechanical buttons, switches, and/or other control elements for providing user input to the endoscopic camera head 100 and/or the system 200 to enable user control over certain functions of the endoscopic camera head 100 and/or the system 200 (e.g., selecting the operating mode, enabling image stabilization, operating lighting and/or imaging capabilities of the endoscopic camera head 100, enabling/permitting compositing of video data streams, rendering processed video to the display 220, etc.). The user interface 216 may include buttons, switches, or other control means disposed on the endoscopic camera head 100 itself independent of or in addition to user interface controls not disposed on the endoscope. Simply as an illustrative example, the endoscopic camera head 100 and/or the display 220 may have input buttons and switches (e.g., the buttons 136A-136B), and, additionally, a keyboard or mouse may be connected directly to the processor 204 (in embodiments where the processor 204 is disposed outside of the endoscopic camera head 100). All of these together constitute the user interface 216.

The display 220 may be or comprise a liquid crystal display (LCD), a light emitting diode (LED) display, a high definition (HD) display, a 4K display, virtual or augmented reality headset, or the like. The display 220 may be a stand-alone display or a display integrated as part of another device, such as a smart phone, a laptop, a tablet, a headset or head-worn device, and/or the like. In one embodiment, the display 220 may be a monitor or other viewing equipment disposed within an operating room, such that video feed captured from a surgery or surgical procedure can be rendered to the display 220 for a physician to view. In some embodiments, the display 220 may comprise a plurality of displays according to, for example, system design.

The network interface 224 may enable one or more components of the system 200 to communicate wired and/or wirelessly with one another or with components outside the system 200. These communication interfaces that permit the components of the system 200 to communicate using the network interface 224 include wired and/or wireless communication interfaces for exchanging data and control signals between one another. Examples of wired communication interfaces/connections include Ethernet connections, HDMI connections, connections that adhere to PCI/PCIe standards and SATA standards, and/or the like. Examples of wireless interfaces/connections include Wi-Fi connections, LTE connections, Bluetooth^{®} connections, NFC connections, and/or the like.

The database 232 includes the same or similar structure as the memory 208 described above. In at least one exemplary embodiment, the database 232 is included in a remote server and stores video data captured during a surgery or surgical procedure (e.g., a camera on an endoscope capturing a live feed during an endoscopy).

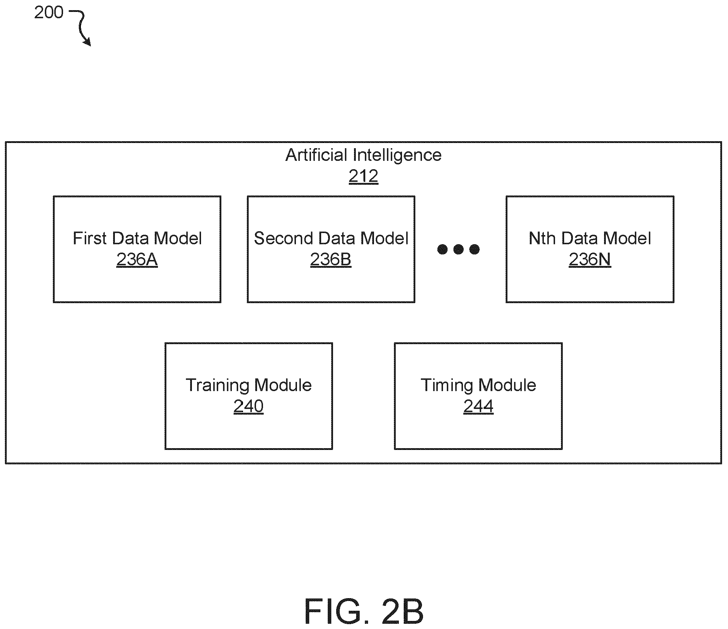

Fig. 2B illustrates aspects of the AI 212 in accordance with at least one embodiment of the present disclosure. The AI 212 includes data models 236A-236N, a training module 240, and a timing module 244. In some embodiments, the AI 212 may comprise additional or alternative components.

The data models 236A-236N may each be or comprise software capable of interacting with the memory 208 to perform one or more functions, or to cause the system 200 or one or more components thereof to perform one or more functions. In some embodiments, the data models 236A-236N may be or comprise AI and/or ML models (e.g., Support Vector Machines (SVMs), Convolutional Neural Networks (CNNs), Deep Neural Networks (DNNs), autoencoders, etc.). The function (or, alternatively, action or corrective action) may be performed during the course of the surgery or surgical procedure to beneficially assist the physician in carrying out the surgery or surgical procedure. For example, the function may be to automatically perform a white balancing when the controller 128 determines that the endoscope 100 is pointed at a white object. As another example, the function may be to orient a surgical image depicting anatomical elements based on identification of other features in the image (e.g., rectilinear lines). In some embodiments, each of the data models 236A-236N may be organized into one or more applications, modules, packages, and/or software layers. Moreover, the number of data models is in no way limited, and different numbers and types of data models may be present in the data models 236A-236N depending on, for example, the type of surgery or surgical procedure, system application, and the like.

Each data model of the data models 236A-236N may perform a different corrective function. For example, the first data model 236A may be trained to perform a white balancing, while the second data model 236B may be trained to orient a surgical scan with gravity (or, more generally, to adjust an image orientation). In other embodiments, the data models 236A-236N may perform different parts of the same action. For example, in white balancing the first data model 236A operates to identify the boundaries of the white object in view of the camera head of the endoscope 100, while the second data model 236B operates to perform the white balancing on the red, green, and blue histograms generated by the image sensors in the camera head. In some embodiments, the data models 236A-236N may be a single data model trained to perform a plurality of different corrective actions during the course of the surgery or surgical procedure.

The training module 240 may be or comprise software and/or data capable of training the one or more of the data models 236A-236N to perform their respective corrective action. The training module 240 may include one or more training sets, which includes data that can be processed by the data models 236A-236N to train the data models 236A-236N. The training data may comprise historical data or information, medical imaging data or information (e.g., preoperative images, intraoperative images, Magnetic Resonance Imaging (MRI) images, Computed Tomography (CT) images, combinations thereof, etc.), and/or other data information that is used to train the data models 236A-236N. For example, the first data model 236A may be trained to identify an anatomical element in a surgical scan, and the training set may comprise similar surgical scans (e.g., of other patients) depicting same type of anatomical element. The training set may be passed into the first data model 236A such that the first data model 236A learns to identify the anatomical element in the surgical scan. In some embodiments, the training module 240 may comprise data training sets that comprise orientation information, which may be collected by the orientation/movement detector 132. The orientation information may be or comprise information about an angle at which an image was captured, information about an angle of the endoscope 100 when capturing the image, pose information of the surgical site or a component thereof such as an anatomical element, combinations thereof, and the like. The orientation information may be passed through one or more of the data models 236A-236N to facilitate training the data models 236A-236N to recognize elements (e.g., anatomical elements, rectilinear lines, etc.) in images and/or a video feed.

The data models 236A-236N may be trained by the training module 240 to perform a variety of corrective actions during the course of the surgery or surgical procedure. For example, the first data model 236A may be trained to detect a boundary of a white object, and the training module 240 may comprise training sets that include data or information about the boundaries of similar white objects. The data or information may be used to train the first data model 236A to detect the white object. For example, the first data model 236A may be or comprise a neural network with a plurality of weights. The data may be used to adjust the weights in the first data model 236A, such that the first data model 236A can determine the boundary of a given white object. In some embodiments, the first data model 236A may be trained to detect the boundary of the white object within a threshold degree of accuracy. Similarly, the second data model 236B may be trained to adjust the light values read by the image sensors when the endoscope 100 is pointed at the white object. The second data model 236B may be trained using light values from previous white balancing with a similar white object, such that the second data model 236B can automatically adjust the white balance of the image produced by the endoscope 100 of the white object. In one embodiment, the second data model 236B may also be or comprise a neural network whose weights are adjusted based on the training data of white balancing of similar white objects.

As another example, the first data model 236A may be trained to orient a surgical image based on an identification of the surrounding environment. In this example, the first data model 236A may be used to identify rectilinear lines in the environment (e.g., the operating room) captured by the camera of the endoscope and classify the image or video feed as having been captured in the operating room. The controller 128 may then use the second data model 236B to rotate or reposition the captured image, such that the image aligns with gravity. In some embodiments, the rotation or reposition may be relative to the body of the patient (e.g., the meniscus, preoperative scans, etc., may be adjusted relative to the body of the patient depicted in the captured image). In other words, if the image were captured at an angle, the second data model 236B may orient the image such that the gravity vector is pointing down when the image is rendered to the display 220. In some embodiments, the second data model 236B may receive information provided by the orientation/movement detector 132 (e.g., a gyroscope, an accelerometer, etc.) to determine the direction of gravity with respect to the endoscope 100 when the image was captured. The second data model 236B may, as a result, know the direction of the gravity vector of the image, and may rotate or reposition the captured image. While white balancing and orienting a surgical image are discussed as examples, these examples are in no way limiting, and the data models 236A-236N may be additionally or alternatively trained to perform other corrective functions. For example, the data models 236A-236N may be trained to focus the endoscope 100, cause an illumination source to emit light, cause the meniscus of the endoscope 100 to be leveled horizontally, cause an imaging device to capture at least one image, cause a repositioning of the imaging device, combinations thereof, and the like. The leveling of the meniscus may occur by rotating the camera around the eye cup of the endoscope. The leveling of the meniscus may enable the user of the endoscope 100 (e.g., a surgeon) to better triangulate surgical tools that appear from the left and right sides of the horizontal meniscus. In other words, by leveling the meniscus, the endoscope 100 may be able to view surgical tools proximate the surgical site in an intuitively understood orientation.

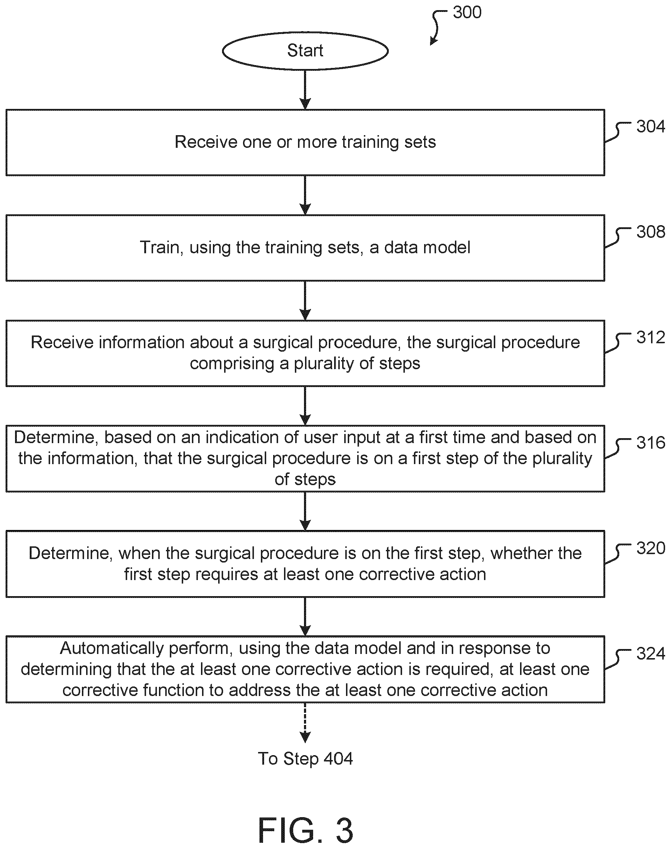

The timing module 244 may be used by the AI 212 to determine a current step of a surgery or surgical procedure. In some embodiments, the timing module 244 may be or comprise software or other data that contains or accesses data from the database related to the surgery or surgical procedure. In some embodiments, the timing module 244 accesses the database 232 to retrieve information about the surgical procedure when prompted by the AI 212. The information about the surgery or surgical procedure may be or comprise information about the number of steps in the surgery or surgical procedure, the amount of time each step takes, combinations thereof, and the like. In some embodiments, the controller 128 and/or the AI 212 may use information provided by the timing module 244 along with the data models 236A-236N to perform one or more corrective actions, as described further in Figs. 3-4 below. For example, the first step in a surgery may be a white balancing, and the timing module 244 may indicate to the controller 128 that the first step is a white balancing, and that the first step takes on average 90 seconds to complete. The AI 212 may use first data model 236A to determine whether the endoscope 100 is pointed at a white object. The first data model 236A may be trained to determine whether an object is white (e.g., whether the average pixel value of the image in view of the endoscope 100 meets or exceeds a certain threshold value) and, if the first data model 236A determines the endoscope 100 is pointed at a white object, the AI 212 may, if the surgery is on the first step, automatically cause the endoscope 100 to perform white balancing, without the physician instructing the system 200 to perform the white balancing.

In some embodiments, the AI 212 is trained using data collected from one or more surgeries or surgical procedures that implement an endoscope (e.g., an endoscopic surgical procedure). The training may comprise providing the AI 212 or specific data models included therein (e.g., the first data model 236A, the second data model 236B, etc.) with the data. In one example, the AI 212 may comprise an autoencoder with an encoder and a decoder. The autoencoder may be used, for example, to denoise an image captured by the endoscope 100.

The autoencoder may receive the training data. The training data may comprise data captured by the endoscope 100 (e.g., images) from previous endoscopic surgical procedures. Additionally or alternatively, the training data may comprise data from other similar endoscopic procedures (e.g., data retrieved from the database 232). The autoencoder may receive the training data and output a reconstruction of the training data. The error between the training data and the reconstruction (e.g., the difference in pixel values when the training data comprise images) may be determined. In some embodiments, the error may be based on a mean squared error, such that the difference between the reconstruction pixel value and the pixel value of the initial data is determined, squared, and summed together, then divided by the total number of pixels in the image. The error may then be backpropagated through the autoencoder. The backpropagation may include determining the contribution of each weight to the overall error and adjusting the weight value using an optimization method, such as gradient descent, to minimize the error. The determination of contribution of each weight may be performed for each layer in the autoencoder. In other words, the error is backpropagated to optimize the autoencoder, such that the overall error between the reconstruction and the initial data decreases with each iteration through the autoencoder. In some embodiments, the training may end (e.g., the autoencoder may be sufficiently trained), when the error to be back propagated falls below a threshold value (e.g., less than 1%, indicating the error in the reconstruction is less than 1%).

In another example, the AI 212 comprises a deep neural network (CNN) trained to learn a correct white balance. For example, the CNN may be trained to receive an image captured by the endoscope 100 and perform a white balancing, such that the output image is white balanced. The CNN may be trained using data captured by the endoscope 100. Additionally or alternatively, the CNN may be trained with data retrieved from the database 232, or using data accessed outside the system 200. The training data may then be passed through the CNN. As the training data passes through the layers of the CNN, each layer caches data needed for the backward propagation (e.g., input values, intermediate values, etc.). The output of the CNN may be compared to the training data, and an error (e.g., a difference between the output and the training data) may be calculated. The error may then be backpropagated through the CNN to adjust the weights of each of the layers. The backpropagation may be used by the CNN to minimize the error, such that subsequent passes of data through the CNN more closely match the training data. In some embodiments, the training may end when the error falls below a threshold value.

Although Figs. 2A-2B illustrate the various elements in the system 200 as being separate from one another, it should be appreciated that some or all of the elements may be integrated with each other if desired. For example, a single desktop or laptop computer may include the processor 204, the memory 208, the user interface 216, and the display 220. It should be further appreciated that each element in the system 200 includes one or more communication interfaces that enable communication with other elements in the system 200 over, for example, the network interface 224. Another example of a preferred embodiment of the system 200 includes an endoscopic camera head 100 with a built in user interface 216 connected to a camera control unit (CCU), the CCU comprising the controller 128, the memory 208, the processor 204, the network interface 224, and a user interface 216, and the CCU is also connected such that it can output image data to the display 220.

Fig. 3 illustrates a method 300 according to at least one exemplary embodiment of the present disclosure. The method 300 may be used, for example, to train and implement a data model in a surgery or surgical procedure.

One or more steps of the method 300 may be carried out or otherwise performed, for example, by at least one processor and/or by a controller. The at least one processor may be the same as or similar to the processor 204 of the system 200 as described above and the controller may be the same as or similar to the controller 128 of the system 200 as described above. A processor other than any processor described herein and/or a controller other than any controller described herein may also be used to execute one or more steps of the method 300. The at least one processor or controller may perform one or more steps of the method 300 by executing elements stored in a memory such as the memory 208. The elements stored on the memory 208 (e.g., instructions and/or other data) and executed by the processor 204 and/or the controller 128 and may cause the processor 204 and/or the controller 128 to execute one or more steps of the method 300.

The method 300 starts and then proceeds to step 304, where one or more training sets are received. The training sets may comprise data that can be used to train a data model (e.g., data models 236A-236N). The method 300 then proceeds to step 308, where a data model (or, in some cases, a plurality of data models) is trained using the training sets. The training sets may be or comprise historical data or information, medical imaging data or information, and/or other data or information. As an example, the first data model 236A may be trained to perform a white balancing by adjusting the light values of the red, blue, and green light captured by image sensors in the endoscope 100 such that the image or video feed rendered to the display 220 is white balanced. Additionally or alternatively, the training sets may comprise orientation data or information, which may be collected and/or stored by an orientation or movement detector.

The method 300 then proceeds to step 312, where information about a surgical procedure is received, the surgical procedure comprising a plurality of steps. The plurality of steps may be predetermined based on the type of surgical procedure, and may be stored in the database 232. The information may also comprise information about the timing of each step of the plurality of steps (e.g., how long each step takes to complete). The method 300 then proceeds to step 316, where the surgical procedure is determined to be on a first step of the plurality of steps based on an indication of user input at a first time and based on the received information. For example, the controller 128 may determine that, based on the physician inputting a command into the system 200 (e.g., via the buttons 136A-136B, via the user interface 216, combinations thereof, etc.), the surgical procedure is on a first step. In some embodiments, the first step may be a white balancing step. In some embodiments, the controller 128 may make the determination that the surgical procedure is on the first step based on information provided by the timing module 244, information rendered to the display 220 (e.g., the video feed is of a white object), combinations thereof, and the like. In one embodiment, the controller 128 may only determine that the surgical procedure is on the first step when the endoscope 100 is pointed at a white object and when a physician provides input into the system 200. While the first step described herein is a white balancing step, the step 312 may determine that the surgical procedure is on another step, such as an imaging step, a step of introducing the endoscope 100 to a surgical site, a step of illuminating the surgical site with the endoscope 100, a step of imaging the surgical site, or the like.

The method 300 then proceeds to step 320 where, when the surgical procedure is on the first step, it is determined whether the first step requires at least one corrective action. Continuing the white balancing example, the controller 128 may determine that white balancing should be performed. In some embodiments, the controller 128 use context (e.g., information collected by the endoscope 100) to determine whether the corrective action is required. For example, the controller 128 may, through use of the AI 212, determine that the readings of the endoscope 100 are inaccurate, and/or that the endoscope 100 has not yet performed a white balancing. In some embodiments, the controller 128 may automatically take corrective action when a task is required for the specific step. For example, the white balancing may be a corrective action that is always performed; in this case, the controller 128 may automatically perform the white balancing when the white balancing step is reached. In other embodiments, the corrective action may be optional, and may only be performed when the controller 128 determines that the corrective action is required. For example, in a step where the endoscope is introduced to the surgical site, the endoscope 100 may be emitting light (e.g., the physician turned the illumination device of the endoscope 100 on before introducing the endoscope 100 to the surgical site). In this case, the controller 128 may determine that a corrective action of illuminating the surgical site is not necessary.

The method 300 then continues to step 324, where at least one corrective function is automatically performed, using the data model and in response to determining that the at least one corrective action is required, to address the at least one corrective action. For example, during the first step of the surgical procedure, the controller 128 may determine that white balancing should be performed, and may access the AI 212 to perform the white balancing. The AI 212 may then use the first data model 236A to perform the white balancing, where the first data model 236A has been trained to adjust the light value readings of the image sensors in the endoscope 100 to white balance the image or video feed generated by the endoscope 100. The method 300 may then end. In some embodiments, the method 300 may repeat. In some embodiments, the method 300 may continue on to step 404 in the method 400, as discussed below.

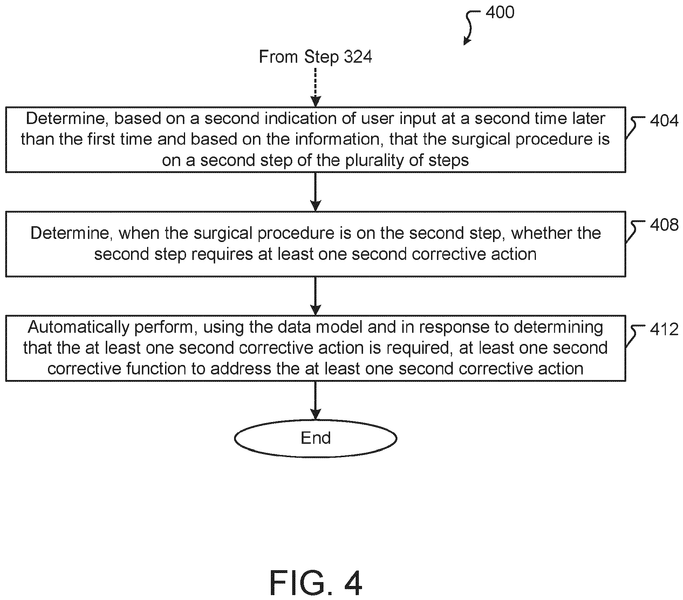

Fig. 4 illustrates a method 400 according to at least one exemplary embodiment of the present disclosure. The method 400 may be used, for example, to implement a data model in a surgery or surgical procedure.

One or more steps of the method 400 may be carried out or otherwise performed, for example, by at least one processor and/or by a controller. The at least one processor may be the same as or similar to the processor 204 of the system 200 as described above and the controller may be the same as or similar to the controller 128 of the system 200 as described above. A processor other than any processor described herein and/or a controller other than any controller described herein may also be used to execute one or more steps of the method 400. The at least one processor or controller may perform one or more steps of the method 400 by executing elements stored in a memory such as the memory 208. The elements stored on the memory 208 (e.g., instructions and/or other data) and executed by the processor 204 and/or the controller 128 and may cause the processor 204 and/or the controller 128 to execute one or more steps of the method 400.

The method 400 may continue from step 324, and may proceed to step 404, where it is determined that, based on a second indication of user input at a second time later than the first time and based on the information, the surgical procedure is on a second step of the plurality of steps. In this case, the physician may provide a command (e.g., via the buttons 136A-136B, via the user interface 216, etc.) that indicates to the controller 128 that the surgical procedure has continued on to the second step in the plurality of steps. For example, the physician may have already performed the white balancing, and may introduce the endoscope 100 to the surgical site. Based on the content of the video feed on the display 220 (e.g., the video feed has changed from a white object to video of the surgical site) and/or timing information from the timing module 244, the controller 128 may determine that the surgical procedure has progressed to the next step. In this example, the second step may comprise the endoscope 100 imaging the surgical site.

The method 400 continues to step 408, where it is determined, when the surgical procedure is on the second step, whether the second step requires at least one second corrective action. As noted above, the second step may be or comprise the endoscope 100 imaging the surgical site, and the second corrective action may be to adjust the illumination device to correctly illuminate the surgical site. The controller 128 may determine whether or not the endoscope 100 is correctly illuminating the surgical site and, when the surgical site is not correctly illuminated, perform the second corrective action of adjusting the illumination device to correctly illuminate the surgical site. For example, the second step of the surgical procedure may be to illuminate the surgical site with a light optimal for ICG fluorescence imaging. The controller 128 may use the AI 212 to analyze the video feed from the endoscope 100 to determine whether the surgical site is illuminated correctly. The AI 212 may use the second data model 236B, for example, to determine whether or not the surgical site is illuminated with light optimal for ICG fluorescence imaging (e.g., illuminated with light with wavelengths between about 750 nm and about 800 nm). If the surgical site is not correctly illuminated, the controller 128 may determine that the second corrective action, such as causing the illumination source to begin emitting light with the correct wavelengths of light to illuminate the surgical scene, should be taken.

The method 400 then proceeds to step 412 where, in response to determining that the at least one second corrective action is required, at least one second corrective function is automatically performed using the data model to address the at least one second corrective action. For example, the controller 128 may automatically enable the illumination source and/or cause the illumination source to emit the light optimal for imaging the surgical site. The method 400 then ends. In some embodiments, the method 400 may be repeated for each step in the plurality of steps of the surgical procedure.

Although several exemplary embodiments have been described with respect to medical procedures that occur internal to a patient, exemplary embodiments may also be applied to medical procedures that generally occur external to a patient.

Any of the steps, functions, and operations discussed herein can be performed continuously and automatically.

While the exemplary embodiments illustrated herein show the various components of the system collocated, certain components of the system can be located remotely, at distant portions of a distributed network, such as a LAN and/or the Internet, or within a dedicated system. Thus, it should be appreciated, that the components of the system can be combined into one or more devices, such as a server, communication device, or collocated on a particular node of a distributed network, such as an analog and/or digital telecommunications network, a packet-switched network, or a circuit-switched network. It will be appreciated from the preceding description, and for reasons of computational efficiency, that the components of the system can be arranged at any location within a distributed network of components without affecting the operation of the system.

Furthermore, it should be appreciated that the various links connecting the elements can be wired or wireless links, or any combination thereof, or any other known or later developed element(s) that is capable of supplying and/or communicating data to and from the connected elements. These wired or wireless links can also be secure links and may be capable of communicating encrypted information. Transmission media used as links, for example, can be any suitable carrier for electrical signals, including coaxial cables, copper wire, and fiber optics, and may take the form of acoustic or light waves, such as those generated during radio-wave and infra-red data communications.

While the flowcharts have been discussed and illustrated in relation to a particular sequence of events, it should be appreciated that changes, additions, and omissions to this sequence can occur without materially affecting the operation of the disclosed embodiments, configuration, and aspects.

A number of variations and modifications of the disclosure can be used. It would be possible to provide for some features of the disclosure without providing others.

In yet another embodiment, the systems and methods of this disclosure can be implemented in conjunction with a special purpose computer, a programmed microprocessor or microcontroller and peripheral integrated circuit element(s), an ASIC or other integrated circuit, a digital signal processor, a hard-wired electronic or logic circuit such as discrete element circuit, a programmable logic device or gate array such as PLD, PLA, FPGA, PAL, special purpose computer, any comparable means, or the like. In general, any device(s) or means capable of implementing the methodology illustrated herein can be used to implement the various aspects of this disclosure. Exemplary hardware that can be used for the present disclosure includes computers, handheld devices, telephones (e.g., cellular, Internet enabled, digital, analog, hybrids, and others), and other hardware known in the art. Some of these devices include processors (e.g., a single or multiple microprocessors), memory, nonvolatile storage, input devices, and output devices. Furthermore, alternative software implementations including, but not limited to, distributed processing or component/object distributed processing, parallel processing, or virtual machine processing can also be constructed to implement the methods described herein.

In yet another embodiment, the disclosed methods may be readily implemented in conjunction with software using object or object-oriented software development environments that provide portable source code that can be used on a variety of computer or workstation platforms. Alternatively, the disclosed system may be implemented partially or fully in hardware using standard logic circuits or VLSI design. Whether software or hardware is used to implement the systems in accordance with this disclosure is dependent on the speed and/or efficiency requirements of the system, the particular function, and the particular software or hardware systems or microprocessor or microcomputer systems being utilized.

In yet another embodiment, the disclosed methods may be partially implemented in software that can be stored on a storage medium, executed on programmed general-purpose computer with the cooperation of a controller and memory, a special purpose computer, a microprocessor, or the like. In these instances, the systems and methods of this disclosure can be implemented as a program embedded on a personal computer such as an applet, JAVA^{®} or CGI script, as a resource residing on a server or computer workstation, as a routine embedded in a dedicated measurement system, system component, or the like. The system can also be implemented by physically incorporating the system and/or method into a software and/or hardware system.

Although the present disclosure describes components and functions implemented in the embodiments with reference to particular standards and protocols, the disclosure is not limited to such standards and protocols. Other similar standards and protocols not mentioned herein are in existence and are considered to be included in the present disclosure. Moreover, the standards and protocols mentioned herein and other similar standards and protocols not mentioned herein are periodically superseded by faster or more effective equivalents having essentially the same functions. Such replacement standards and protocols having the same functions are considered equivalents included in the present disclosure.

The present disclosure, in various embodiments, configurations, and aspects, includes components, methods, processes, systems and/or apparatus substantially as depicted and described herein, including various embodiments, sub-combinations, and subsets thereof. Those of skill in the art will understand how to make and use the systems and methods disclosed herein after understanding the present disclosure. The present disclosure, in various embodiments, configurations, and aspects, includes providing devices and processes in the absence of items not depicted and/or described herein or in various embodiments, configurations, or aspects hereof, including in the absence of such items as may have been used in previous devices or processes, e.g., for improving performance, achieving ease, and/or reducing cost of implementation.

The foregoing discussion of the disclosure has been presented for purposes of illustration and description. The foregoing is not intended to limit the disclosure to the form or forms disclosed herein. In the foregoing Detailed Description for example, various features of the disclosure are grouped together in one or more embodiments, configurations, or aspects for the purpose of streamlining the disclosure. The features of the embodiments, configurations, or aspects of the disclosure may be combined in alternate embodiments, configurations, or aspects other than those discussed above. This method of disclosure is not to be interpreted as reflecting an intention that the claimed disclosure requires more features than are expressly recited in each claim. Rather, as the following claims reflect, inventive aspects lie in less than all features of a single foregoing disclosed embodiment, configuration, or aspect. Thus, the following claims are hereby incorporated into this Detailed Description, with each claim standing on its own as a separate preferred embodiment of the disclosure.

Moreover, though the description of the disclosure has included description of one or more embodiments, configurations, or aspects and certain variations and modifications, other variations, combinations, and modifications are within the scope of the disclosure, e.g., as may be within the skill and knowledge of those in the art, after understanding the present disclosure. It is intended to obtain rights, which include alternative embodiments, configurations, or aspects to the extent permitted, including alternate, interchangeable and/or equivalent structures, functions, ranges, or steps to those claimed, whether or not such alternate, interchangeable and/or equivalent structures, functions, ranges, or steps are disclosed herein, and without intending to publicly dedicate any patentable subject matter.

Example aspects of the present disclosure include:
A surgical system for adjusting an endoscope according to at least one embodiment of the present disclosure comprises: a processor; and a memory storing instructions thereon that, when processed by the processor, cause the processor to: receive information about an endoscopic surgical procedure, the endoscopic surgical procedure comprising a plurality of steps; determine, based on an indication of user input at a first time and based on the information, that the endoscopic surgical procedure is on a first step of the plurality of steps; determine, when the endoscopic surgical procedure is on the first step, whether the first step requires at least one corrective action to address at least one of an image captured by the endoscope and a function of the endoscope during the first step; and automatically perform, using a data model and in response to determining that the at least one corrective action is required, at least one corrective function during the first step to address the at least one corrective action.

Any of the aspects herein, wherein the at least one corrective function is at least one of performing a white balancing, adjusting an image orientation, focusing a surgical scope, causing an illumination source to emit light, causing an imaging device to capture at least one image, and causing a repositioning of the imaging device.

Any of the aspects herein, wherein the instructions further cause the processor to: train, using one or more training sets, the data model to perform the at least one corrective function.

Any of the aspects herein, wherein the one or more training sets comprises image data.

Any of the aspects herein, wherein the image data comprise one or more preoperative images, one or more intraoperative images, one or more magnetic resonance imaging (MRI) images, one or more computed tomography (CT) images, or a combination thereof.

Any of the aspects herein, wherein the one or more training sets comprises orientation information collected by an orientation detector.

Any of the aspects herein, wherein the instructions further cause the processor to: determine, based on a second indication of user input at a second time later than the first time and based on the information, that the endoscopic surgical procedure is on a second step of the plurality of steps; determine, when the endoscopic surgical procedure is on the second step, whether the second step requires at least one second corrective action to address at least one of a second image captured by the endoscope and a second function of the endoscope during the second step; and automatically perform, using the data model and in response to determining that the at least one second corrective action is required, at least one second corrective function during the second step to address the at least one second corrective action.

Any of the aspects herein, wherein the at least one second corrective function comprises at least one of performing a white balancing, adjusting an image orientation, focusing a surgical scope, causing an illumination source to emit light, causing an imaging device to capture at least one image, and causing a repositioning of the imaging device.

An apparatus according to at least one embodiment of the present disclosure comprises: an endoscope or exoscope; a processor; and a memory storing instructions thereon that, when processed by the processor, cause the processor to: receive information about an endoscopic surgical procedure, the endoscopic surgical procedure comprising a plurality of steps; determine, based on an indication of user input at a first time and based on the information, that the endoscopic surgical procedure is on a first step of the plurality of steps; determine, when the endoscopic surgical procedure is on the first step, whether the first step requires at least one corrective action to address at least one of an image captured by the endoscope and a function of the endoscope during the first step; and automatically perform, using a data model and in response to determining that the at least one corrective action is required, at least one corrective function during the first step to address the at least one corrective action.

Any of the aspects herein, wherein the at least one corrective function is at least one of performing a white balancing, adjusting an image orientation, focusing a surgical scope, causing an illumination source to emit light, causing an imaging device to capture at least one image, and causing a repositioning of the imaging device.

Any of the aspects herein, wherein the instructions further cause the processor to: train, using one or more training sets, the data model to perform the at least one corrective function.

Any of the aspects herein, wherein the one or more training sets comprises image data.

Any of the aspects herein, wherein the image data comprise one or more preoperative images, one or more intraoperative images, one or more magnetic resonance imaging (MRI) images, one or more computed tomography (CT) images, or a combination thereof.

Any of the aspects herein, wherein the one or more training sets comprises orientation information collected by an orientation detector.

Any of the aspects herein, wherein the instructions further cause the processor to: determine, based on a second indication of user input at a second time later than the first time and based on the information, that the endoscopic surgical procedure is on a second step of the plurality of steps; determine, when the endoscopic surgical procedure is on the second step, whether the second step requires at least one second corrective action to address at least one of a second image captured by the endoscope and a second function of the endoscope during the second step; and automatically perform, using the data model and in response to determining that the at least one second corrective action is required, at least one second corrective function during the second step to address the at least one second corrective action.

Any of the aspects herein, wherein the at least one second corrective function comprises at least one of performing a white balancing, adjusting an image orientation, focusing a surgical scope, causing an illumination source to emit light, causing an imaging device to capture at least one image, and causing a repositioning of the imaging device.

A method for adjusting an endoscope according to at least one embodiment of the present disclosure comprises: receiving information about an endoscopic surgical procedure, the endoscopic surgical procedure comprising a plurality of steps; determining, based on an indication of user input at a first time and based on the information, that the endoscopic surgical procedure is on a first step of the plurality of steps; determining, when the endoscopic surgical procedure is on the first step, whether the first step requires at least one corrective action to address at least one of an image captured by the endoscope and a function of the endoscope during the first step; and automatically performing, using a data model and in response to determining that the at least one corrective action is required, at least one corrective function during the first step to address the at least one corrective action.

Any of the aspects herein, wherein the at least one corrective function is at least one of performing a white balancing, adjusting an image orientation, focusing a surgical scope, causing an illumination source to emit light, causing an imaging device to capture at least one image, and causing a repositioning of the imaging device.

Any of the aspects herein, further comprising: training, using one or more training sets, the data model to perform the at least one corrective function.

Any of the aspects herein, further comprising: determining, based on a second indication of user input at a second time later than the first time and based on the information, that the endoscopic surgical procedure is on a second step of the plurality of steps; determining, when the endoscopic surgical procedure is on the second step, whether the second step requires at least one second corrective action to address at least one of a second image captured by the endoscope and a second function of the endoscope during the second step; and automatically performing, using the data model and in response to determining that the at least one second corrective action is required, at least one second corrective function during the second step to address the at least one second corrective action.

Any aspect in combination with any one or more other aspects,
Any one or more of the features disclosed herein,
Any one or more of the features as substantially disclosed herein,
Any one or more of the features as substantially disclosed herein in combination with any one or more other features as substantially disclosed herein.

Any one of the aspects/features/embodiments in combination with any one or more other aspects/feature/embodiments.

Use of any one or more of the aspects or features as disclosed herein.

It is to be appreciated that any feature described herein can be claimed in combination with any other feature(s) as described herein, regardless of whether the features come from the same described embodiment.

The phrases "at least one," "one or more," "or," and "and/or" are open-ended expressions that are both conjunctive and disjunctive in operation. For example, each of the expressions "at least one of A, B and C," "at least one of A, B, or C," "one or more of A, B, and C," "one or more of A, B, or C," "A, B, and/or C," and "A, B, or C" means A alone, B alone, C alone, A and B together, A and C together, B and C together, or A, B and C together.

The term "a" or "an" entity refers to one or more of that entity. As such, the terms "a" (or "an"), "one or more," and "at least one" can be used interchangeably herein. It is also to be noted that the terms "comprising," "including," and "having" can be used interchangeably.

Aspects of the present disclosure may take the form of an embodiment that is entirely hardware, an embodiment that is entirely software (including firmware, resident software, micro-code, etc.) or an embodiment combining software and hardware aspects that may all generally be referred to herein as a "circuit," "module," or "system." Any combination of one or more computer-readable medium(s) may be utilized. The computer-readable medium may be a computer-readable signal medium or a computer-readable storage medium.

A computer-readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer-readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer-readable storage medium may be any tangible medium that can contain or store a program for use by or in connection with an instruction execution system, apparatus, or device.

The terms "determine," "calculate," "compute," and variations thereof, as used herein, are used interchangeably and include any type of methodology, process, mathematical operation or technique.

## Claims

1. A surgical system for adjusting an endoscope, the system comprising:
a processor; and
a memory storing instructions thereon that, when processed by the processor, cause the processor to:
receive information about an endoscopic surgical procedure, the endoscopic surgical procedure comprising a plurality of steps;
determine, based on an indication of user input at a first time and based on the information, that the endoscopic surgical procedure is on a first step of the plurality of steps;
determine, when the endoscopic surgical procedure is on the first step, whether the first step requires at least one corrective action to address at least one of an image captured by the endoscope and a function of the endoscope during the first step; and
automatically perform, using a data model and in response to determining that the at least one corrective action is required, at least one corrective function during the first step to address the at least one corrective action.

2. The surgical system of claim 1, wherein the at least one corrective function is at least one of performing a white balancing, adjusting an image orientation, focusing a surgical scope, causing an illumination source to emit light, causing an imaging device to capture at least one image, and causing a repositioning of the imaging device.

3. The surgical system of claim 1, wherein the instructions further cause the processor to:
train, using one or more training sets, the data model to perform the at least one corrective function.

4. The surgical system of claim 3, wherein the one or more training sets comprises image data.

5. The surgical system of claim 4, wherein the image data comprise one or more preoperative images, one or more intraoperative images, one or more magnetic resonance imaging (MRI) images, one or more computed tomography (CT) images, or a combination thereof.

6. The surgical system of claim 3, wherein the one or more training sets comprises orientation information collected by an orientation detector.

7. The surgical system of claim 1, wherein the instructions further cause the processor to:
determine, based on a second indication of user input at a second time later than the first time and based on the information, that the endoscopic surgical procedure is on a second step of the plurality of steps;
determine, when the endoscopic surgical procedure is on the second step, whether the second step requires at least one second corrective action to address at least one of a second image captured by the endoscope and a second function of the endoscope during the second step; and
automatically perform, using the data model and in response to determining that the at least one second corrective action is required, at least one second corrective function during the second step to address the at least one second corrective action.

8. The surgical system of claim 7, wherein the at least one second corrective function comprises at least one of performing a white balancing, adjusting an image orientation, focusing a surgical scope, causing an illumination source to emit light, causing an imaging device to capture at least one image, and causing a repositioning of the imaging device.

9. An apparatus, comprising:
an endoscope or exoscope;
a processor; and
a memory storing instructions thereon that, when processed by the processor, cause the processor to:
receive information about an endoscopic surgical procedure, the endoscopic surgical procedure comprising a plurality of steps;
determine, based on an indication of user input at a first time and based on the information, that the endoscopic surgical procedure is on a first step of the plurality of steps;
determine, when the endoscopic surgical procedure is on the first step, whether the first step requires at least one corrective action to address at least one of an image captured by the endoscope and a function of the endoscope during the first step; and
automatically perform, using a data model and in response to determining that the at least one corrective action is required, at least one corrective function during the first step to address the at least one corrective action.

10. The apparatus of claim 9, wherein the at least one corrective function is at least one of performing a white balancing, adjusting an image orientation, focusing a surgical scope, causing an illumination source to emit light, causing an imaging device to capture at least one image, and causing a repositioning of the imaging device.

11. The apparatus of claim 9, wherein the instructions further cause the processor to:
train, using one or more training sets, the data model to perform the at least one corrective function.

12. The apparatus of claim 11, wherein the one or more training sets comprises image data.

13. The apparatus of claim 12, wherein the image data comprise one or more preoperative images, one or more intraoperative images, one or more magnetic resonance imaging (MRI) images, one or more computed tomography (CT) images, or a combination thereof.

14. The apparatus of claim 11, wherein the one or more training sets comprises orientation information collected by an orientation detector.

15. The apparatus of claim 9, wherein the instructions further cause the processor to:
determine, based on a second indication of user input at a second time later than the first time and based on the information, that the endoscopic surgical procedure is on a second step of the plurality of steps;
determine, when the endoscopic surgical procedure is on the second step, whether the second step requires at least one second corrective action to address at least one of a second image captured by the endoscope and a second function of the endoscope during the second step; and
automatically perform, using the data model and in response to determining that the at least one second corrective action is required, at least one second corrective function during the second step to address the at least one second corrective action.

16. The apparatus of claim 15, wherein the at least one second corrective function comprises at least one of performing a white balancing, adjusting an image orientation, focusing a surgical scope, causing an illumination source to emit light, causing an imaging device to capture at least one image, and causing a repositioning of the imaging device.

17. A method for adjusting an endoscope, comprising:
receiving information about an endoscopic surgical procedure, the endoscopic surgical procedure comprising a plurality of steps;
determining, based on an indication of user input at a first time and based on the information, that the endoscopic surgical procedure is on a first step of the plurality of steps;
determining, when the endoscopic surgical procedure is on the first step, whether the first step requires at least one corrective action to address at least one of an image captured by the endoscope and a function of the endoscope during the first step; and
automatically performing, using a data model and in response to determining that the at least one corrective action is required, at least one corrective function during the first step to address the at least one corrective action.

18. The method of claim 17, wherein the at least one corrective function is at least one of performing a white balancing, adjusting an image orientation, focusing a surgical scope, causing an illumination source to emit light, causing an imaging device to capture at least one image, and causing a repositioning of the imaging device.

19. The method of claim 17, further comprising:
training, using one or more training sets, the data model to perform the at least one corrective function.

20. The method of claim 17, further comprising:
determining, based on a second indication of user input at a second time later than the first time and based on the information, that the endoscopic surgical procedure is on a second step of the plurality of steps;
determining, when the endoscopic surgical procedure is on the second step, whether the second step requires at least one second corrective action to address at least one of a second image captured by the endoscope and a second function of the endoscope during the second step; and
automatically performing, using the data model and in response to determining that the at least one second corrective action is required, at least one second corrective function during the second step to address the at least one second corrective action.
